# EUROPEAN PATENT SPECIFICATION

(11) **EP 4 388 259 B1**
(45) Date of publication and mention of the grant of the patent: **15.07.2026**
(21) Application number: 21953617.4
(22) Date of filing: 25.08.2021
(51) Int. Cl.: F26B 7/00, A01G 7/00, F26B 23/08, F26B 25/22, F26B 5/04, A23B 2/10

(54) **METHODS AND APPARATUS FOR DRYING CANNABIS**
VERFAHREN UND VORRICHTUNG ZUM TROCKNEN VON CANNABIS
PROCÉDÉS ET APPAREIL DE SÉCHAGE DE CANNABIS

(30) Priority: 17.08.2021 US 202163234074 P
(43) Date of publication of application: 26.06.2024
(73) Proprietor: EnWave Corporation, Delta, BC V3M 6R9 (CA)
(72) Inventor: ZHANG, Guopeng, Delta, BC V3M 6R9 (CA); AHMAD, Shafique, Delta, BC V3M 6R9 (CA); SUCU, Mehmet, Delta, BC V3M 6R9 (CA)
(74) Representative: Porta & Consulenti Associati S.p.A.
(86) International application number: PCT/CA2021/051180
(87) International publication number: WO 2023/019343

(56) References cited:
- WO-A1-2020/223784
- WO-A1-2020/223784
- CA-A- 1 223 496
- CA-A1- 3 070 605
- CN-A- 109 470 046
- CN-B- 102 524 903
- GB-A- 2 141 319
- US-A1- 2016 258 680
- US-A1- 2020 178 572
- US-A1- 2020 246 495
- US-B2- 9 879 908

## Description

### Field of the Invention

The invention pertains to methods of drying cannabis using microwave vacuum drying.

### Background of the Invention

Terpenes are naturally occurring, volatile organic compounds present in cannabis and many other plants. The beneficial properties of some essential oils are derived from terpenes. In cannabis, terpenes are responsible for the particular odour and flavour of the different cannabis strains. Different terpenes are present in varying amounts in the various cannabis strains. Cannabis strains have particular terpene profile expressions that are important to consumers, and no two strains have the same terpene profile.

Terpenes begin to evaporate at just above room temperature. Conventional cannabis drying methods do not usually cause substantial degradation of cannabinoids, which are quite stable and non-volatile. However, terpenes are volatile and there is significant loss when cannabis is dried by conventional methods. Maximizing terpene retention is recognized in the industry as being important in the production of premium quality cannabis products.

The most common method used to dry cannabis is a slow room drying process which is generally referred to as "hang drying" or "rack drying." In these methods, cannabis branches are either hung up or placed on racks. In another conventional method, the trimmed flowers are placed in trays in a single layer and the trays are put onto racks in a drying room. In general, these conventional drying methods can take from about five to fifteen days, depending on the size of the branches and buds, and the surrounding environment. Lengthy cannabis drying results in the loss of lighter terpenes. Monoterpenes are the most volatile as they have the lowest evaporation points and dissipate first during drying, even before water.

WO 2019/041017 (EnWave Corporation) discloses drying cannabis using a microwave vacuum drying apparatus that both pasteurizes and dries the cannabis in a single operation. However, pasteurization requires relatively high temperatures and a sufficient time of exposure to microwave radiation, so the method is not suitable where the goal is preserve terpenes to the greatest extent possible.

Also WO 2020/223784 A1 discloses a microwave vacuum drying apparatus according to the prior art.

It would be desirable to provide a process the drying cannabis while maximizing the retention of terpenes, especially the more volatile monoterpenes and sesquiterpenes, producing a dried product that retains more terpenes than using the prior art methods.

### Summary of the Invention

The invention is a method according to claim 1 and an apparatus according claim 13.

According to the invention, the method is a method of drying cannabis in a vacuum chamber having a plurality of adjacent drying zones, the drying zones comprising a first drying zone adjacent to an input end of the vacuum chamber, a last drying zone adjacent to an output end of the vacuum chamber, and one or more drying zones arranged adjacent to each other between the first drying zone and the last drying zone, the vacuum chamber being at a pressure less than atmospheric, comprising:
(a) loading the cannabis into the vacuum chamber;
(b) conveying the cannabis from an input end of the vacuum chamber to an output end through each of the drying zones between the input end and the output end while exposing the cannabis to microwave radiation;
   (i) each of the drying zones having a microwave power level different from the microwave power level of an immediately adjacent drying zone,
   (ii) the microwave power levels of respective drying zones are set such that, for each drying zone between the first drying zone and the last drying zone, the power level is either lower than the power level of the two immediately adjacent drying zones, or higher than the power level of the two immediately adjacent drying zones; and
(c) unloading the dried cannabis from the vacuum chamber.

According to the invention, the apparatus is an apparatus for dehydrating cannabis, comprising:
(a) a vacuum chamber having a plurality of adjacent drying zones, the drying zones comprising a first drying zone adjacent to an input end of the vacuum chamber, a last drying zone adjacent to an output end of the vacuum chamber, and one or more drying zones between the first drying zone and the last drying zone;
(b) means for loading the cannabis into the vacuum chamber at the input end;
(c) means for unloading the cannabis from the vacuum chamber at the output end;
(d) means for conveying the cannabis from the input end through the plurality of drying zones to the output end; and
(e) microwave generators having a microwave power level in each of the drying zones,
which is characterized in that the microwave power level of each drying zone between the first drying zone and the last drying zone is either (i) lower than in both immediately adjacent drying zones or (ii) higher than in both immediately adjacent drying zones.

Further aspects of the invention and features of specific embodiments of the invention are described below.

### Brief Description of the Drawings

Figure 1 is a schematic drawing of a continuous throughput microwave vacuum apparatus for carrying out one embodiment of the method of the invention.
Figure 2 is a graph showing the surface temperature of cannabis flowers during the continuous throughput drying process of Example 1.
Figure 3 is a graph showing the surface temperature of cannabis flowers during the batch drying process of Example 2.
Figure 4 is a flowchart of a method for batch drying of cannabis according to another embodiment of the invention.

### Detailed Description

The invention provides methods for drying cannabis in a microwave vacuum drying apparatus, while maximizing the retention of terpenes.

### Continuous Throughput Drying

For drying in a continuous throughput process, a microwave vacuum chamber is provided having a plurality of drying zones, each of which has its microwave power set at a selected power level. The power levels are set in an alternating arrangement such that, as the cannabis is moved through the vacuum chamber, its maximum temperature is maintained at a target temperature that is optimal for drying the cannabis while maximizing terpene retention.

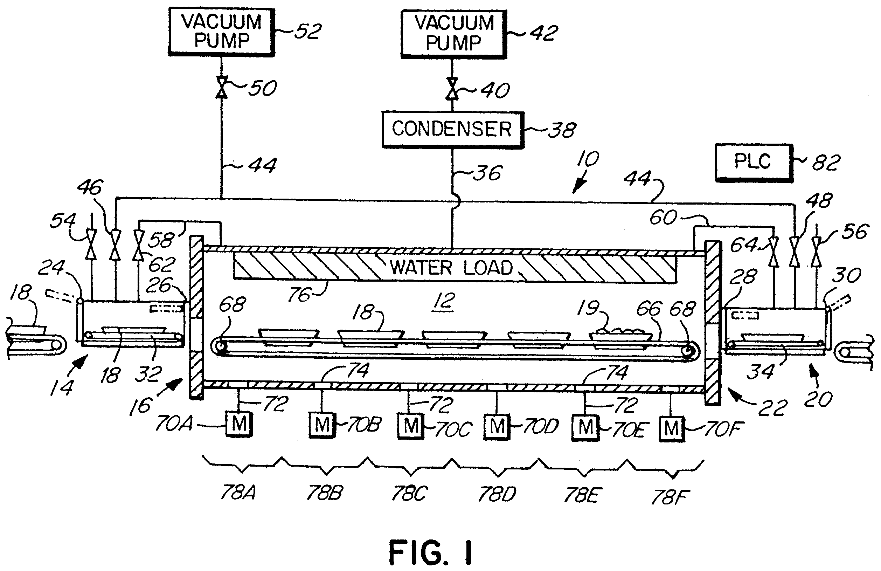

Figure 1 schematically illustrates an embodiment of the continuous throughput microwave vacuum drying apparatus 10. It has a vacuum chamber **12** through which a tray of cannabis is conveyed for dehydration. A loading module **14** is positioned at the input end **16** of the vacuum chamber for introduction of trays **18** of cannabis material 19 into the vacuum chamber 12. A discharge module **20** is positioned at the output or discharge end **22** of the vacuum chamber for removal of the trays of cannabis. The loading module **14** and discharge module **20** each have a pair of airlock doors, respectively **24, 26** and **28, 30** (their open position being shown by dotted lines in Figure 1). These permit the trays to be loaded into and unloaded from the vacuum chamber, while maintaining the vacuum chamber at the reduced pressure required for the dehydration process. The loading and discharge modules **14, 20** have motor-driven conveyors **32, 34,** respectively, for moving the trays.

The vacuum chamber **12** is connected via a vacuum pipe **36,** a condenser **38** and a shut-off valve **40** to a vacuum pump **42** or the vacuum system of a plant. The loading and discharge modules **14, 20** are connected via a vacuum pipe **44** and shut-off valves **46, 48** and **50** to a vacuum pump 52. The loading and discharge modules are vented by discharge shut-off valves **54** and **56** respectively. A further discharge valve (not shown) is provided for venting the vacuum chamber. The loading and discharge modules **14, 20** are connected to the vacuum chamber **12** for pressure equalization by means of equalization conduits **58** and **60** and the associated shut-off valves **62** and **64,** respectively.

The vacuum chamber **12** has a motor-driven conveyor **66** extending longitudinally through it and arranged to support and convey the trays 18. The conveyor runs on rollers **68** adjacent to the inlet and the outlet ends of the vacuum chamber.

Microwave generators or groups of microwave generators **70A to 70F** are mounted below the vacuum chamber **12** and are arranged to radiate microwave energy into the vacuum chamber through waveguides **72** and microwave-transparent windows 74. Each microwave generator group **70** comprises one or more microwave generators, for example six or eight microwave generators.

The microwave generators are connected to a conventional power source (not shown) which provides the required electric power. The microwave generators are cooled by coolant pumped to circulate around them from a cooling liquid refrigeration unit. A water load **76** is provided at the upper part of the vacuum chamber **12** to absorb microwave energy and thus prevent reflection of microwaves in the vacuum chamber. The water is pumped through tubing by a water load pump.

The vacuum chamber 12 has six drying zones **78A, 78B, 78C, 78D, 78E** and **78F,** each of which has a respective group of microwave generators **70A** to **70F.** The first drying zone 78A is adjacent to the input end 16 of the vacuum chamber, the last drying zone 78F is adjacent to the output end 22, and the other four drying zones are arranged adjacent to each other between the first and last drying zones. In other embodiments, the vacuum chamber may comprise more or fewer than six drying zones, for example one drying zone or four drying zones or 12 drying zones. In the illustrated embodiment each of the six drying zones extends approximately one-sixth of the length of the vacuum chamber. For example, in a vacuum chamber that is 4.2 meters in length, each of the drying zones is about 0.7 meters long.

The vacuum chamber zone 12 is not partitioned into separate pressure zones and accordingly the six drying zones **78A** to **78F** are at the same vacuum pressure. The distinction between the zones is the level of microwave power that is applied in each zone. This may be accomplished, for example, by the number of microwave generators or generator groups 70 in each drying zone, or by means of higher power microwave generators in a given zone, or by setting the microwave generators at a particular power level in a given zone. In the illustrated embodiment, each microwave generator group may have a power output in the range of 0.2 to 10 kW.

The dehydration apparatus 10 includes a programmable logic controller (PLC) 82, programmed and connected to control the operation of the system, including the pressure and temperature sensors, the conveyor drive motors, the airlock doors, the microwave generators, the water load pump, the vacuum pumps, the condenser, the refrigerant pump and the vacuum shut-off valves.

It will be understood that the invention applies to microwave vacuum dehydrators having any of various means for feeding, conveying and discharging the cannabis material. In one embodiment, as illustrated, the cannabis is transported in trays which ride on a conveyor belt, with feeding into and discharge from the vacuum chamber being done by means of loading and discharge modules having airlock doors. In another embodiment, the cannabis material is fed into and out of the vacuum chamber by means of rotary vacuum valves rather than airlocks with doors, and is transported directly on the conveyor belt rather than on trays. In yet another embodiment, the material is transported through the vacuum chamber in a rotating basket, an arrangement of the type disclosed in WO 2009/049409 to EnWave Corporation. The present invention is independent of any particular means for moving the cannabis material into, through and out of the vacuum chamber.

The basic steps of the method of drying are as follows. The vacuum pressure is set to the desired pressure, for example, a pressure in the range of about 10 to 50 Torr (13 to 67 mbar), alternatively about 20 to 30 Torr (27 to 40 mbar), alternatively about 25 Torr (33 mbar). The microwave generators are activated at the power levels selected for each of the drying zones. The cannabis material is loaded into the vacuum chamber. This is done on a continuous throughput basis and the material is conveyed at constant speed through each of the drying zones in the vacuum chamber. It is irradiated with microwave energy in each drying zone at the microwave power level of the respective drying zone. After the last drying zone, the dried cannabis is unloaded from the vacuum chamber.

The method of the invention may be carried out using the dehydration apparatus **10.** The airlock doors **26** and **30** are closed and the vacuum pumps, water load pump, conveyor drive motors and microwave generators are actuated, all under the control of the PLC **82.** Pressure within the vacuum chamber is reduced to the desired vacuum pressure for drying. The cannabis **19** to be dehydrated is put into a tray **18** and the tray is placed in the loading module **14.** The outer airlock door **24** and shut-off valve **52** are closed and the loading module is evacuated by the vacuum pump **45** to the pressure of the vacuum chamber. The inner airlock door **26** is then opened and the tray is transported, by the conveyors **32** and **66,** into the vacuum chamber **12.** Once the tray is fully inside the vacuum chamber, the loading chamber **14** is prepared for receiving a second tray, by closing the inner airlock door **26** and the shut-off valves **46** and **62,** opening the shut-off valve **54** to vent the loading module to atmospheric pressure, and opening the outer airlock door **24.** The dehydration apparatus is thus able to process multiple trays of cannabis material at the same time, in a continuous process. Inside the vacuum chamber **12,** the tray is moved along the conveyor **66** through each of the drying zones **78A** to **78F** and the microwave generator groups **70A to 70F** irradiate the cannabis and dehydrate it. Moisture given off by the cannabis is conveyed to the condenser **40** to be condensed to liquid water. The residence time in the vacuum chamber, as the cannabis is moved at constant speed from the input end to the discharge end, is about 70 minutes, alternatively in the range of 50 to 90 minutes, alternatively in the range of 80 to 150 minutes. The length of each drying zone **78A** to **78E** is approximately equal in this embodiment, and as the conveyor **66** moves the cannabis at a constant speed, the residence time in each drying zone is the same. The target maximum temperature of the cannabis during treatment is about 40°C, or alternatively about 39°C, or in the range of 30 to 45°C. The cannabis is dried to a desired moisture content, for example in the range of 1 to 16 wt.%, alternatively in the range of 2 to 14 wt.%. The drying process retains a high proportion of the terpenes in the cannabis, for example at least 75 wt.%, or at least 80 wt.%, or at least 89 wt.%.

At the end of the residence time within the vacuum chamber, the tray **18 of** cannabis enters the discharge module **20,** where it is conveyed toward the outer airlock door **30.** The inner airlock door **28** is then closed, the shut-off valves **48, 64** are closed, the valve **56** is opened to vent the discharge module to the atmosphere, the outer airlock door **30** is opened and the tray is removed. The discharge module is prepared for the next tray of fresh cannabis to be removed from the vacuum chamber by closing the outer airlock door **30,** evacuating the discharge module by means of vacuum pump **52** to the reduced pressure of the vacuum chamber, and opening the inner airlock door **28.** Following either loading or discharge of a tray from the loading module or discharge module, the vacuum pump **52** draws gases from the loading or discharge module, through the vacuum conduit **64,** without disturbing the vacuum in the vacuum chamber **12.**

The microwave power levels of the drying zones are set so that as a tray of cannabis is conveyed, at constant speed, through the vacuum chamber from the input end to the output end, it is exposed to alternating higher and lower microwave power levels. This maintains the maximum temperature of the cannabis within a narrow range about the target temperature. For example, in the embodiment of Figure 1, having six drying zones, the power levels may be set at about 8.0 -10 kW in the first drying zone **78A,** 4.0-5.0 kW in the second drying zone **78B,** 5.5-7.5 kW in the third drying zone **78C,** 1.6-3.0 kW in the fourth drying zone **78D,** 3.0-5.0 kW in the fifth drying zone **78E,** and 0-3.0 kW in the sixth (last) drying zone **78F.** The power settings are such that, for each drying zone between the first drying zone **78A** and the last drying zone **78F,** the power level is either lower than the power level of the two immediately adjacent drying zones, or higher than the power level of the two immediately adjacent drying zones. Thus, the microwave power levels do not ascend or descend in a linear manner, i.e., they do not simply increase or decrease from the first drying zone to the last drying zone. Rather, the power levels of adjacent drying zones alternate between relatively higher and relatively lower power levels. The alternating cooling of the product in a lower power level drying zone and heating of the product in a higher power level drying zone (all within the desired range about the target temperature) dries the product efficiently. Power levels and residence times may be set having regard to the weight of cannabis being dried.

### Batch Drying

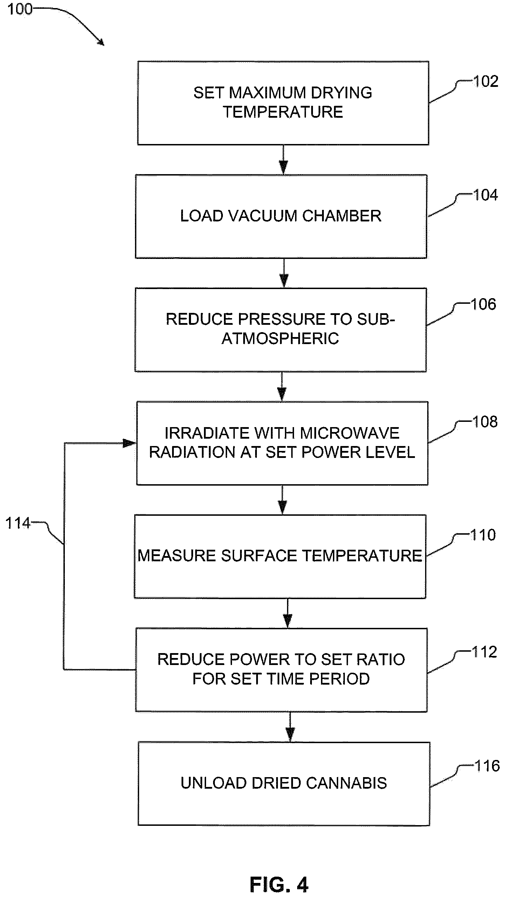

An embodiment of the invention further provides a method of batch drying of cannabis that maximizes terpene retention. In this embodiment, fresh cannabis is dried in a batch-type microwave vacuum dryer, for example a resonance cavity microwave vacuum dryer. This drying method 100 is depicted in the flowchart of Figure 4. The maximum surface temperature at which the cannabis is to be dried is set **102** at a selected temperature, for example 40°C, or a temperature in the range of 30-45°C. This temperature is selected to be sufficient low to minimize the loss of terpenes during drying while still being high enough to dry the cannabis within an acceptably short time period. This target temperature corresponds to a set power level. Fresh cannabis is loaded **104** into the microwave vacuum dryer. The pressure is reduced **106** to a pressure less than atmospheric, for example a pressure in the range of 10-50 Torr (13-67 mbar), alternatively about 20-30 Torr (27-40 mbar), alternatively about 25 Torr (33 mbar). The cannabis is irradiated 108 with microwave radiation at a set power level. The surface temperature of the cannabis is measured 110. When a temperature sensor reads a temperature value that is higher than the selected temperature for a specified time period, the microwave power level is reduced **112** to a specified ratio of the original power level, and to stay at that reduced power level for a specified time period before returning to the original level.

This control of the microwave power level may be done automatically by means of programming of a PLC that operates the drying apparatus. The specified time for the temperature to be higher than the selected temperature may, for example, be in the range of 1 and 60 seconds; the specified ratio of the reduced microwave power level to the original power level may be in the range of 5 and 95%; the specified time period for the power level to be reduced before returning to the original power level may be in the range of 5 to 600 seconds. Steps **108, 110** and **112** are then repeated **114.** In a typical process, these three steps are repeated many times during the drying period, which may be, for example, in the range of 70-150 minutes. In this manner, the selected maximum surface temperature of the cannabis is maintained during the drying process. When the cannabis has been dried to the desired extent, the dried cannabis is unloaded **116** from the microwave vacuum chamber.

In some embodiments of the batch drying process, the total energy applied during the process is allocated to different power levels. For example, the drying may be done at two or more descending power levels, such as two or more of power levels 6.0 kW, 4.5 kW, 3 kW, 2 kW and 1.5 kW.

### Examples

### Example 1 - Continuous Throughput Drying

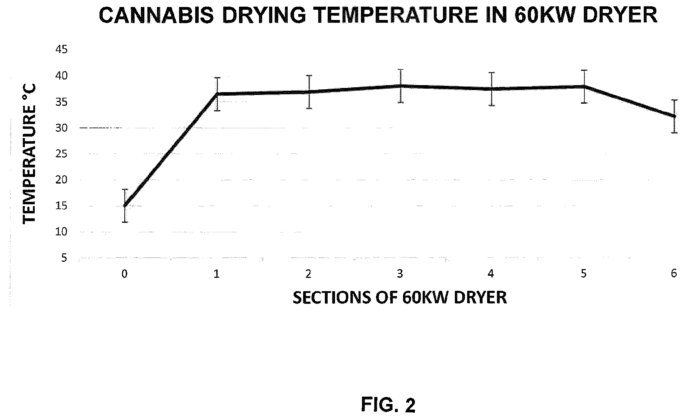

Freshly trimmed cannabis flowers of the "LA Confidential" strain were loaded in multilayer fashion in polyethylene drying trays, with a loading depth of about 2.5 inches. The total product weight for each tray was 5.0 kg. The microwave vacuum drying process was carried out using a 60 kW dryer marketed by EnWave Corporation under the brand QuantaREV. The filled trays were fed automatically on a conveyor belt to the entrance vacuum lock of the dryer. The pressure of the vacuum chamber was 20 Torr. The filled trays travelled horizontally on a conveyor belt inside the chamber, passing through the six drying zones. The microwave power levels for the drying zones were set at alternating high, medium and low values, specifically, the power settings for the six drying sections, from the input end to the output end, were 10 kW, 4.4 kW, 7.4 kW, 1.6 kW, 5.0 kW and 1.6 kW, respectively. The total power applied was 30 kW and the total residence time (i.e., the drying time in the vacuum chamber) for each filled tray was about 70 minutes. About every 8 minutes one tray with fresh cannabis entered into the dryer at the input side and at the same time, one tray with dried cannabis exited from the output side. The hourly throughput of the process was around 37 kg of fresh cannabis. Figure 2 shows the temperature of the cannabis in each of the six drying zones. The maximum surface temperature of the cannabis was approximately 39°C.

Total terpene measurements were done on the dried cannabis produced by this process, as well as on fresh cannabis material and on dried cannabis from a control example done by conventional rack drying. The rack drying was done for time periods in the range of 7-10 days, at temperatures in the range of 18-21°C, and at an ambient humidity in the range of 55-65%. The materials were cryogenically ground, extracted and subjected to gas chromatography and mass spectrometry. Triplicate measurements were done on each extracted sample. The total terpene retention was 88.862 wt.% for the cannabis dried by the continuous throughput microwave vacuum drying, whereas it was 74.313 wt.% for the cannabis dried by conventional rack drying.

The example described above was repeated using a different strain of cannabis, namely the "Think Fast" strain. Table 1 shows the total terpene concentration on a dry basis of both the LA Confidential strain and the Think Fast strain, in comparison with the respective rack drying control examples.

**Table 1**

| **Strain** | **Terpene Concentration (wt.%) - Microwave Vacuum Drying** | **Terpene Concentration (wt.%) - Room Drying** | **% Greater Retention with Microwave Vacuum Drying** |
|---|---|---|---|
| LA Confidential | 1.742 | 1.457 | 19.56 |
| Think Fast | 2.233 | 1.509 | 47.98 |

Compared to the room dried control cannabis flowers from the same harvest, the microwave vacuum dried LA Confidential strain flowers contained 19.56% more terpenes and the Think Fast strain cannabis flowers retained 47.98% more terpenes.

### Example 2 - Batch Drying

Trimmed fresh cannabis flowers of the "Kush Hemp" strain were dried using a 10 kW resonance cavity microwave vacuum dryer marketed by EnWave Corporation under the brand NUTRAREV. The cannabis was loaded in multilayer fashion in polyethylene drying trays, with a loading depth of approximately 2.0 inches. The total batch weight was 8.0 kg, comprising 1.0 kg on each of 8 trays.

The maximum surface temperature for the cannabis was set at 40°C on the PLC screen. When the temperature sensor read a temperature value that was higher than 40°C for a time of 2 seconds, the microwave power level was reduced automatically by the PLC to 50% of the set power level, and to stay at that reduced power level for 2 seconds before returning to the original level.

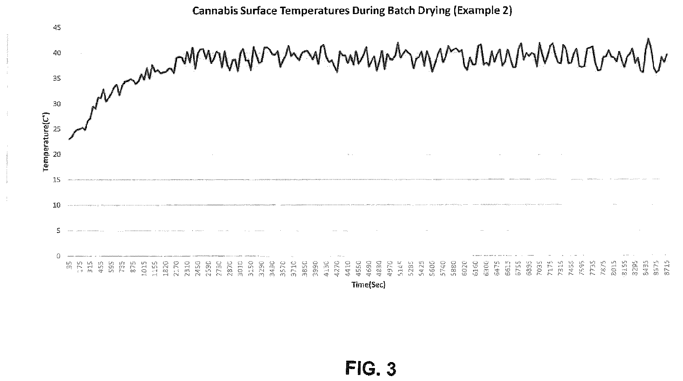

The initial moisture content of the cannabis flowers was 81 wt.% and the target residual moisture content after drying was 16 wt.%. The target moisture loss was calculated at 6.19 kg. Based on an empirical drying rate of 1.05 kg/kWh, the total required microwave energy needed was 5.90 kWh. In this example, the total energy was allocated to three descending power levels, namely, 5000 W, 3500 W and 2000 W, for 1.965 kWh at each level. The total drying time was about 2.5 hours. The surface temperature profile of the cannabis during the drying process is shown in Figure 3. The temperature of the cannabis increased gradually from around 20°C and then remained steady at slightly over 40°C during the 2.5 hour drying time.

Total terpene measurements were done as described for Example 1 on the dried cannabis produced by this batch process, as well as on fresh cannabis and on dried cannabis from a control example done by conventional rack drying. The terpene concentration was 2.51 wt.% for the cannabis dried was by the microwave vacuum process and 1.87 wt.% for the control example. Compared to the room-dried control cannabis flowers from the same harvest, the microwave vacuum-dried cannabis contained 34.58% more terpenes.

Throughout the foregoing description and the drawings, specific details have been set forth in order to provide a more thorough understanding to persons skilled in the art. However, well known elements may not have been shown or described in detail to avoid unnecessarily obscuring the disclosure. Accordingly, the description and drawings are to be regarded in an illustrative, rather than a restrictive, sense.

Accordingly, the scope of the invention is to be construed in accordance with the following claims.

## Claims

1. A method of drying cannabis in a vacuum chamber (12) having a plurality of adjacent drying zones (78A-78F), the drying zones comprising a first drying zone (78A) adjacent to an input end (16) of the vacuum chamber (12), a last drying zone (78F) adjacent to an output end (22) of the vacuum chamber (12), and one or more drying zones (78B-78E) arranged adjacent to each other between the first drying zone (78A) and the last drying zone (78F), the vacuum chamber being at a pressure less than atmospheric, comprising:
(a) loading the cannabis (19) into the vacuum chamber;
(b) conveying the cannabis from an input end (16) of the vacuum chamber to an output end (22) through each of the drying zones between the input end and the output end while exposing the cannabis to microwave radiation;
(i) each of the drying zones having a microwave power level different from the microwave power level of an immediately adjacent drying zone,
(ii) the microwave power levels of respective drying zones are set such that, for each drying zone between the first drying zone (78A) and the last drying zone (78F), the power level is either lower than the power level of the two immediately adjacent drying zones, or higher than the power level of the two immediately adjacent drying zones; and
(c) unloading the dried cannabis from the vacuum chamber.

2. The method of claim 1, wherein the cannabis is conveyed through two or more drying zones between the first drying zone and the last drying zone.

3. The method of any one of the preceding claims, wherein the cannabis is conveyed through three or more drying zones between the first drying zone and the last drying zone.

4. The method of any one of the preceding claims, wherein the conveying of the cannabis through the vacuum chamber is done at constant speed.

5. The method of any one of the preceding claims, wherein the drying zones are of equal length.

6. The method of any one of the preceding claims, wherein an average surface temperature of the cannabis in the vacuum chamber is maintained at 40°C or less.

7. The method of any one of claims 1-6, wherein an average surface temperature of the cannabis in the vacuum chamber is maintained at in the range of 30-45°C.

8. The method of any one of the preceding claims, wherein the pressure is in the range of 10-50 Torr.

9. The method of any one of the preceding claims, wherein the pressure is in the range of 20-30 Torr.

10. The method of any one of the preceding claims, wherein a residence time of the cannabis in the vacuum chamber is in the range of 80-150 minutes, alternatively in the range of 50-90 minutes.

11. The method of any one of the preceding claims, wherein the cannabis is dried to a moisture content in the range of 1-16 wt.%.

12. The method of any one of the preceding claims, wherein the cannabis to be dried contains terpenes and after drying the dried cannabis contains at least 75 wt.% of said terpenes, alternatively at least 80 wt.% of said terpenes, alternatively at least 89 wt.% of said terpenes.

13. An apparatus (10) for dehydrating cannabis, comprising:
(a) a vacuum chamber (12) having a plurality of adjacent drying zones (78), the drying zones comprising a first drying zone (78A) adjacent to an input end (16) of the vacuum chamber, a last drying zone (78F) adjacent to an output end (22) of the vacuum chamber, and one or more drying zones (78B, 78C, 78D, 78E) between the first drying zone and the last drying zone;
(b) means (14) for loading the cannabis into the vacuum chamber at the input end;
(c) means (20) for unloading the cannabis from the vacuum chamber at the output end;
(d) means (66,68) for conveying the cannabis from the input end through the plurality of drying zones to the output end; and
(e) microwave generators (70A-70F) having a microwave power level in each of the drying zones (78),
**characterized in that** the microwave power level of each drying zone (78B, 78C, 78D, 78E) between the first drying zone (78A) and the last drying zone (78F) is either (i) lower than in both immediately adjacent drying zones or (ii) higher than in both immediately adjacent drying zones.

14. The apparatus of claim 13, comprising two or more said drying zones (78B, 78C, 78D, 78E) between the first drying zone (78A) and the last drying zone (78F).

## Patentansprüche

1. Verfahren zum Trocknen von Cannabis in einer Vakuumkammer (12) mit einer Vielzahl von benachbarten Trocknungszonen (78A-78F), wobei die Trocknungszonen eine erste Trocknungszone (78A), die neben einem Eingangsende (16) der Vakuumkammer (12), eine letzte Trocknungszone (78F), die neben einem Ausgangsende (22) der Vakuumkammer (12) und eine oder mehrere Trocknungszonen (78B-78E), die nebeneinander zwischen der ersten Trocknungszone (78A) und der letzten Trocknungszone (78F) angeordnet sind, umfassen, wobei in der Vakuumkammer ein Druck herrscht, der geringer ist als der Atmosphärendruck, umfassend:
(a) Laden des Cannabis (19) in die Vakuumkammer;
(b) Transportieren des Cannabis von einem Eingangsende (16) der Vakuumkammer zu einem Ausgangsende (22) durch jede der Trocknungszonen zwischen dem Eingangsende und dem Ausgangsende, während das Cannabis einer Mikrowellenstrahlung ausgesetzt wird;
(i) wobei jede der Trocknungszonen einen Mikrowellenleistungspegel aufweist, der sich von dem Mikrowellenleistungspegel einer unmittelbar benachbarten Trocknungszone unterscheidet,
(ii) die Mikrowellenleistungspegel der jeweiligen Trocknungszonen so eingestellt werden, dass für jede Trocknungszone zwischen der ersten Trocknungszone (78A) und der letzten Trocknungszone (78F) der Leistungspegel entweder niedriger als der Leistungspegel der beiden unmittelbar benachbarten Trocknungszonen oder höher als der Leistungspegel der beiden unmittelbar benachbarten Trocknungszonen ist; und
(c) Entladen des getrockneten Cannabis aus der Vakuumkammer.

2. Verfahren nach Anspruch 1, wobei das Cannabis zwischen der ersten Trocknungszone und der letzten Trocknungszone durch zwei oder mehr Trocknungszonen transportiert wird.

3. Verfahren nach einem der vorstehenden Ansprüche, wobei das Cannabis zwischen der ersten Trocknungszone und der letzten Trocknungszone durch drei oder mehr Trocknungszonen transportiert wird.

4. Verfahren nach einem der vorstehenden Ansprüche, wobei das Transportieren des Cannabis durch die Vakuumkammer mit konstanter Geschwindigkeit erfolgt.

5. Verfahren nach einem der vorstehenden Ansprüche, wobei die Trocknungszonen gleich lang sind.

6. Verfahren nach einem der vorstehenden Ansprüche, wobei die durchschnittliche Oberflächentemperatur des Cannabis in der Vakuumkammer bei 40 °C oder weniger gehalten wird.

7. Verfahren nach einem der Ansprüche 1 bis 6, wobei eine durchschnittliche Oberflächentemperatur des Cannabis in der Vakuumkammer im Bereich von 30 bis 45 °C gehalten wird.

8. Verfahren nach einem der vorstehenden Ansprüche, wobei der Druck im Bereich von 10 bis 50 Torr liegt.

9. Das Verfahren nach einem der vorhergehenden Ansprüche, wobei der Druck im Bereich von 20 bis 30 Torr liegt.

10. Verfahren nach einem der vorstehenden Ansprüche, wobei die Verweilzeit des Cannabis in der Vakuumkammer im Bereich von 80 bis150 Minuten, alternativ im Bereich von 50 bis 90 Minuten liegt.

11. Verfahren nach einem der vorstehenden Ansprüche, wobei das Cannabis auf einen Feuchtigkeitsgehalt im Bereich von 1 bis 16 Gew.-% getrocknet wird.

12. Verfahren nach einem der vorstehenden Ansprüche, wobei das zu trocknende Cannabis Terpene enthält und das getrocknete Cannabis nach dem Trocknen mindestens 75 Gew.-% der Terpene, alternativ mindestens 80 Gew.-% der Terpene, alternativ mindestens 89 Gew.-% der Terpene enthält.

13. Vorrichtung (10) zur Dehydrierung von Cannabis, umfassend:
(a) eine Vakuumkammer (12) mit einer Vielzahl von benachbarten Trocknungszonen (78), wobei die Trocknungszonen eine erste Trocknungszone (78A) neben einem Eingangsende (16) der Vakuumkammer, eine letzte Trocknungszone (78F) neben einem Ausgangsende (22) der Vakuumkammer und eine oder mehrere Trocknungszonen (78B, 78C, 78D, 78E) zwischen der ersten Trocknungszone und der letzten Trocknungszone umfassen;
(b) Mittel (14) zum Laden des Cannabis in die Vakuumkammer am Eingangsende;
(c) Mittel (20) zum Entladen des Cannabis aus der Vakuumkammer am Auslassende;
(d) Mittel (66, 68) zum Transportieren des Cannabis vom Eingangsende durch die Vielzahl von Trocknungszonen zum Ausgangsende; und
(e) Mikrowellengeneratoren (70A-70F) mit einem Mikrowellenleistungspegel in jeder der Trocknungszonen (78),
**dadurch gekennzeichnet, dass** der Mikrowellenleistungspegel jeder Trocknungszone (78B, 78C, 78D, 78E) zwischen der ersten Trocknungszone (78A) und der letzten Trocknungszone (78F) entweder (i) niedriger als in den beiden unmittelbar benachbarten Trocknungszonen oder (ii) höher als in den beiden unmittelbar benachbarten Trocknungszonen ist.

14. Vorrichtung nach Anspruch 13, umfassend zwei oder mehr der Trocknungszonen (78B, 78C, 78D, 78E) zwischen der ersten Trocknungszone (78A) und der letzten Trocknungszone (78F).

## Revendications

1. Procédé de séchage de cannabis dans une chambre à vide (12) comportant une pluralité de zones de séchage adjacentes (78A-78F), les zones de séchage comprenant une première zone de séchage (78A) adjacente à une extrémité d'entrée (16) de la chambre à vide (12), une dernière zone de séchage (78F) adjacente à une extrémité de sortie (22) de la chambre à vide (12), et une ou plusieurs zones de séchage (78B-78E) disposées adjacentes l'une à l'autre entre la première zone de séchage (78A) et la dernière zone de séchage (78F), la chambre à vide étant à une pression inférieure à la pression atmosphérique, comprenant :
(a) le chargement du cannabis (19) dans la chambre à vide ;
(b) le transport du cannabis d'une extrémité d'entrée (16) de la chambre à vide à une extrémité de sortie (22) à travers chacune des zones de séchage entre l'extrémité d'entrée et l'extrémité de sortie tout en exposant le cannabis à un rayonnement micro-ondes ;
(i) chacune des zones de séchage présentant un niveau de puissance de micro-ondes différent du niveau de puissance de micro-ondes d'une zone de séchage immédiatement adjacente,
(ii) les niveaux de puissance de micro-ondes des zones de séchage respectives sont réglés de telle sorte que, pour chaque zone de séchage entre la première zone de séchage (78A) et la dernière zone de séchage (78F), le niveau de puissance est soit inférieur au niveau de puissance des deux zones de séchage immédiatement adjacentes, soit supérieur au niveau de puissance des deux zones de séchage immédiatement adjacentes ; et
(c) le déchargement du cannabis séché de la chambre à vide.

2. Procédé selon la revendication 1, dans lequel le cannabis est transporté à travers deux zones de séchage ou plus entre la première zone de séchage et la dernière zone de séchage.

3. Procédé selon l'une quelconque des revendications précédentes, dans lequel le cannabis est transporté à travers trois zones de séchage ou plus entre la première zone de séchage et la dernière zone de séchage.

4. Procédé selon l'une quelconque des revendications précédentes, dans lequel le transport du cannabis à travers la chambre à vide est effectué à une vitesse constante.

5. Procédé selon l'une quelconque des revendications précédentes, dans lequel les zones de séchage sont de longueur égale.

6. Procédé selon l'une des revendications précédentes, dans lequel une température moyenne de surface du cannabis dans la chambre à vide est maintenue à 40 °C ou moins.

7. Procédé selon l'une quelconque des revendications 1 à 6, dans lequel la température moyenne de surface du cannabis dans la chambre à vide est maintenue dans la plage de 30 à 45 °C.

8. Procédé selon l'une quelconque des revendications précédentes, dans lequel la pression est dans la plage de 10 à 50 Torr.

9. Procédé selon l'une quelconque des revendications précédentes, dans lequel la pression est dans la plage de 20 à 30 Torr.

10. Procédé selon l'une quelconque des revendications précédentes, dans lequel un temps de séjour du cannabis dans la chambre à vide est dans la plage de 80 à 150 minutes, en variante dans la plage de 50 à 90 minutes.

11. Procédé selon l'une quelconque des revendications précédentes, dans lequel le cannabis est séché jusqu'à un taux d'humidité dans la plage de 1 à 16 % en poids.

12. Procédé selon l'une quelconque des revendications précédentes, dans lequel le cannabis à sécher contient des terpènes et, après séchage, le cannabis séché contient au moins 75 % en poids desdits terpènes, en variante au moins 80 % en poids desdits terpènes, en variante au moins 89 % en poids desdits terpènes.

13. Appareil (10) pour la déshydratation de cannabis, comprenant :
(a) une chambre à vide (12) comportant une pluralité de zones de séchage adjacentes (78), les zones de séchage comprenant une première zone de séchage (78A) adjacente à une extrémité d'entrée (16) de la chambre à vide, une dernière zone de séchage (78F) adjacente à une extrémité de sortie (22) de la chambre à vide, et une ou plusieurs zones de séchage (78B, 78C, 78D, 78E) entre la première zone de séchage et la dernière zone de séchage ;
(b) des moyens (14) pour charger le cannabis dans la chambre à vide à l'extrémité d'entrée ;
(c) des moyens (20) pour décharger le cannabis de la chambre à vide à l'extrémité de sortie ;
(d) des moyens (66,68) pour transporter le cannabis de l'extrémité d'entrée à l'extrémité de sortie en passant par la pluralité de zones de séchage ; et
(e) des générateurs de micro-ondes (70A-70F) présentant un niveau de puissance de micro-ondes dans chacune des zones de séchage (78),
**caractérisé en ce que** le niveau de puissance de micro-ondes de chaque zone de séchage (78B, 78C, 78D, 78E) entre la première zone de séchage (78A) et la dernière zone de séchage (78F) est soit (i) inférieur à celui des deux zones de séchage immédiatement adjacentes, soit (ii) supérieur à celui des deux zones de séchage immédiatement adjacentes.

14. Appareil selon la revendication 13, comprenant deux dites zones de séchage ou plus (78B, 78C, 78D, 78E) entre la première zone de séchage (78A) et la dernière zone de séchage (78F).
